# DEMANDE DE BREVET EUROPEEN

(11) **EP 4 283 298 A1**
(43) Date de publication de la demande: **29.11.2023**
(21) Numéro de dépôt: 23175398.9
(22) Date de dépôt: 25.05.2023
(51) Int. Cl.: G01N 33/569, A01N 25/34, C07K 19/00, C09D 189/00

(54) **BIOCAPTEUR COMPRENANT UNE PLURALITE D' ENSEMBLES D' ATOMES DE CARBONE A L' ETAT D' HYBRIDATION SP2 FONCTIONNALISÉS, SON PROCEDE DE PREPARATION ET SON UTILISATION, NOTAMMENT POUR LA DETECTION DE RISQUES ECO-TOXICOLOGIQUES**

(30) Priorité: 25.05.2022 FR 2205063
(71) Demandeur: Commissariat à l'énergie atomique et aux énergies alternatives, 75015 Paris (FR)
(72) Inventeur: EYVRARD, Doriane, 38054 Grenoble cedex 09 (FR); ALAVA, Thomas, 38054 Grenoble cedex 09 (FR)
(74) Mandataire: Gevers & Orès

(57) **Abrégé**

La présente invention concerne un biocapteur comprenant une pluralité d'ensembles d'atomes de carbone à l'état d'hybridation sp² fonctionnalisés, son procédé de préparation et son utilisation, notamment pour la détection de risques écotoxicologiques, en particulier la détermination de la toxicité de substances présentes dans un milieu aqueux, ainsi que le procédé de détermination correspondant.

## Description

La présente invention concerne un biocapteur comprenant une pluralité d'ensembles d'atomes de carbone à l'état d'hybridation sp² fonctionnalisés, son procédé de préparation et son utilisation, notamment pour la détection de risques écotoxicologiques, en particulier la détermination de la toxicité de substances présentes dans un milieu aqueux, ainsi que le procédé de détermination correspondant.

Les impacts potentiels de l'omniprésence de contaminants dans l'environnement du fait de l'urbanisation, de l'agriculture et des activités industrielles sur la santé des populations constituent une préoccupation importante en santé publique.

Une analyse régulière doit ainsi être mise en oeuvre pour contrôler les niveaux de contamination et d'écotoxicité, et l'évaluation de ces impacts représente un défi de taille pour la société.

La méthodologie de l'évaluation du risque écotoxicologique a été développée à partir des années 80 en tant qu'outil permettant d'apprécier les impacts de l'exposition aux contaminants de l'environnement sur la santé des populations. Cette méthodologie a été utilisée pour évaluer la sécurité des produits et les impacts de nouveaux projets industriels, pour établir des critères et des normes ou pour valider certains niveaux d'exposition permis par règlements.

Traditionnellement, la méthodologie de l'évaluation des risques comprend les quatre étapes suivantes : l'identification du danger, la définition des relations dose-réponse, l'évaluation de l'exposition ciblée, et la caractérisation du risque.

Cette évaluation des risques est basée sur des tests réalisés en laboratoire sur différents modèles biologiques/biochimiques, pour différents temps d'exposition (courts ou longs, pour l'évaluation d'une éventuelle toxicité aiguë ou chronique).

Cependant, ces risques sont très probablement sous-estimés car l'effet cocktail, qui peut générer des toxicités plus importantes que les molécules étudiées isolément, n'est pas évalué à une échelle réaliste. En effet, l'effet cocktail est actuellement évalué en laboratoire en étudiant la dangerosité d'un ensemble connu de molécules exogènes, ou en appliquant des algorithmes à un ensemble de concentrations toxiques mesurées en laboratoire sur des substances étudiées séparément. Dans ce cadre, l'ensemble des molécules réellement présentes dans le milieu naturel ne peut être considéré.

En effet, un effet cocktail se produit notamment lorsqu'une substance chimique, que l'on croyait inoffensive à faible dose, devient nocive à cette même dose, voire à des doses inférieures, si elle est présente en mélange avec au moins une autre. Sa toxicité est potentialisée par l'action de l'autre substance.

Ainsi, les méthodologies mentionnées ci-dessus ne permettent pas de savoir quelles sont les interactions entre plusieurs substances, quels sont les effets lorsque l'on est exposé à plusieurs substances en même temps. Or cette situation est généralement la règle dans le milieu naturel : les organismes vivants et les êtres humains sont en permanence exposés à de multiples substances différentes : médicaments, pesticides, perturbateurs endocriniens, polluants, contaminants, composés organiques volatiles, les produits secondaires et sous-produits correspondants.

Pour comprendre tous les effets possibles de toutes ces substances, il faudrait donc être capable d'observer leurs effets simultanés sur un organisme.

Pour ce faire, ont été développées plus récemment des méthodes de détection in situ des effets écotoxiques induits par des polluants, sur des organismes aquatiques modèles en écotoxicologie.

Ces méthodes concernent notamment l'analyse de la qualité d'un milieu aquatique à l'aide d'outil de biosurveillance recourant à l'exposition de populations de gammares pour notamment identifier l'existence ou non d'une écotoxicité liée à la présence de polluants dans les milieux, son niveau d'intensité, et son évolution dans le temps.

Toutefois, ces méthodes ne permettent pas l'automatisation de l'acquisition des résultats.

En outre, les temps d'obtention de ces résultats sont excessivement longs car impliquent de nombreuses étapes chronophages (élevage des gammares, sélection, installation sur site, récupération et analyses en laboratoire, etc.).

L'invention a pour but de permettre la mise en oeuvre d'un biocapteur permettant une détection et une évaluation des risques éco-toxicologiques, par exemple dans les milieux naturels et les eaux industrielles, qui évite les inconvénients précités.

Ainsi, un but de l'invention est de fournir une méthode in situ permettant l'obtention rapide de résultats, tout en permettant un gain d'espace nécessaire à l'obtention de ces résultats.

Un autre but de l'invention est de fournir une méthode permettant l'obtention de résultats mesurés en temps réel, par des moyens automatisables et miniaturisables.

Ainsi, selon un premier aspect, l'invention concerne un biocapteur comprenant :
- un premier ensemble A d'atomes de carbone à l'état d'hybridation sp², ledit ensemble étant en contact avec :
   ∘ une pluralité de composés de formule (I₁) suivante :

      (R-L₁-W)ₙ-V-L₂-X-Y (I₁),

      et
   ∘ éventuellement, une pluralité de composés de formule (II₁) suivante :

      (R-L₁-W)ₙ-V-L₂-X-Z (II₁),
- un deuxième ensemble B d'atomes de carbone à l'état d'hybridation sp², ledit ensemble étant en contact avec :
   ∘ une pluralité de composés de formule (I₂) suivante :

      (R-L₁-W)ₙ-V-L₂-X-Y (I₂),

      et
   ∘ une pluralité de composés de formule (II₂) suivante :

      (R-L₁-W)ₙ-V-L₂-X-Z (II₂),
- au moins un troisième ensemble C d'atomes de carbone à l'état d'hybridation sp², ledit ensemble étant en contact avec :
   ∘ une pluralité de composés de formule (I₃) suivante :

      (R-L₁-W)ₙ-V-L₂-X-Y (I₃),

      et
   ∘ une pluralité de composés de formule (II₃) suivante :

      (R-L₁-W)ₙ-V-L₂-X-Z (II₃),

      avec, indépendamment de l'ensemble de carbone A à C et du composé de formule (I) à (II) :
      n est égal à 1, 2, ou 3, notamment 3 ;
      V représente, pour tout n, -C(H) ₃₋ₙ- ou encore:
         - lorsque n est égal à 2, -C(H)=, C étant dans ce cas lié à L₂ par une liaison double ;
         - lorsque n est égal à 1, -C=, C étant dans ce cas lié à L₂ par une liaison triple ;
      R est un hydrocarbure aromatique comprenant de 2 à 6 cycles aromatiques condensés; L₁ un groupe C₁-C₁₂ alcane diyle ;
      W représente une liaison simple, un groupe arène diyle, un groupe hétéroarène diyle ou un groupe -O-Ar₁- où Ar₁ est un arène ou un hétéroarène ;
      L₂ est un groupe de formule (A) suivante -(L₂ₐ)ᵢ-(L_{2b})ⱼ- dans laquelle :
         i et j sont indépendamment l'un de l'autre choisis parmi 0 et 1, avec i + j = 1 ou 2 ;
         L₂ₐ est un groupe C₁-C₁₂ alcane diyle, C₂-C₁₂ alcène diyle ou C₂-C₁₂ alcyne diyle ;
         L_{2b} est un groupe arène diyle, un groupe hétéroarène diyle ou un groupe -O-
         Ar₂- où Ar₂ est un arène ou un hétéroarène ;
      X est un groupe -C(=O)N-, -C(=O)O- ou -C(=O)S- ;
      Y est un anticorps antibactérien ou une lectine;
      Z est un peptide antibactérien ;
et dans lequel les ensembles A, B et C sont tels que :
- l'ensemble A est dénué de composés de formule (II₁) ou le rapport molaire de la quantité de composés de formule (II₁) sur celle de composés de formule (I₁) est inférieur à 0,1;
- dans l'ensemble B, le rapport molaire de la quantité de composés de formule (I₂) sur celle de composés de formule (II₂) étant compris de 0,66 à 1,5, par exemple environ 1,0 ; et
- dans l'ensemble C, le rapport molaire de la quantité de composés de formule (I₃) sur celle de composés de formule (II₃) étant compris de 0,33 à 0,66, par exemple environ 0,5.

Ainsi, dans les deux formules (I) et (II) (c'est-à-dire les formules (I₁), (II₁), (I₂), (II₂), (I₃), et (II₃)), C représente un atome de carbone tétravalent dans le cas où V représente -C(H)₃₋ₙ- (-C-, -CH-, ou -CH₂- suivant la valeur de n); un atome de carbone trivalent lorsque V représente - C(H)= ; ou un atome de carbone divalent lorsque V représente -C=.

Par « en contact », on entend notamment que les hydrocarbures aromatiques (R) des composés de formule (I) et (II) forment avec l'ensemble d'atomes de carbone à l'état d'hybridation sp² des liaisons de type empilement-Pi. Ces liaisons, appelées également empilement π- π ou Pi-stacking en anglais, sont des liaisons non covalentes, parmi les plus fortes des liaisons non covalentes.

Selon un mode de réalisation particulier, le biocapteur selon l'invention comprend au moins deux ensembles C d'atomes de carbone à l'état d'hybridation sp², pour lesquels le rapport molaire de la quantité de composés de formule (I₃) sur celle de composés de formule (II₃), étant compris de 0,33 à 0,66, diffère.

Selon un mode de réalisation particulier, le biocapteur selon l'invention comprend au moins deux ensembles B d'atomes de carbone à l'état d'hybridation sp², pour lesquels le rapport molaire de la quantité de composés de formule (I₂) sur celle de composés de formule (II₂), étant compris de 0,66 à 1,5, diffère.

Selon un mode de réalisation particulier, le biocapteur selon l'invention comprend au moins deux ensembles B d'atomes de carbone à l'état d'hybridation sp², pour lesquels le rapport molaire de la quantité de composés de formule (I₂) sur celle de composés de formule (II₂), étant compris de 0,66 à 1,5, diffère et comprenant au moins deux ensembles C d'atomes de carbone à l'état d'hybridation sp², pour lesquels le rapport molaire de la quantité de composés de formule (I₃) sur celle de composés de formule (II₃), étant compris de 0,33 à 0,66, diffère.

Selon un mode de réalisation particulier, lesdits ensembles A, B et C d'atomes de carbone à l'état d'hybridation sp² sont indépendamment choisis parmi le graphène, l'oxyde de graphène, le graphite, les nanotubes de carbones, les fullerènes et les fullerites, lesdits ensembles étant en particulier du graphène, plus particulièrement sous la forme de feuille ou de flocons.

Par « feuille », on entend notamment du graphène, en particulier mono-couche, dont deux des dimensions sont supérieures à 1 mm.

Par « flocon », on entend notamment du graphène, en particulier ayant de 1 à 10 couches, par exemple mono-couche, dont l'une voire deux des dimensions est(sont) inférieure(s) ou égale(s) à 1 mm, en particulier 500µm, 100 µm ou 10 µm.

Selon un mode de réalisation particulier, lesdits ensembles A, B et C d'atomes de carbone à l'état d'hybridation sp² sont de même nature, en particulier du graphène, plus particulièrement sous la forme de feuille ou de flocons.

Selon un mode de réalisation particulier :
- l'ensemble B d'atomes de carbone à l'état d'hybridation sp² est du graphène sous la forme de feuille, et/ou
- l'ensemble C d'atomes de carbone à l'état d'hybridation sp² est du graphène sous la forme de flocons.

Selon un mode de réalisation particulier, l'invention concerne un biocapteur comprenant :
- un premier ensemble A d'atomes de carbone à l'état d'hybridation sp², ledit ensemble étant en contact avec :
   ∘ une pluralité de composés de formule (I₁) suivante :

      (Py-L₁-W)₃-C-L₂-X-Y (I₁),

      et
   ∘ éventuellement, une pluralité de composés de formule (II₁) suivante :

      (Py-L₁-W)₃-C-L₂-X-Z (II₁),
- un deuxième ensemble B d'atomes de carbone à l'état d'hybridation sp², ledit ensemble étant en contact avec :
   ∘ une pluralité de composés de formule (I₂) suivante :

      (Py-L₁-W)₃-C-L₂-X-Y (I₂),

      et
   ∘ une pluralité de composés de formule (II₂) suivante :

      (Py-L₁-W)₃-C-L₂-X-Z (II₂),
- au moins un troisième ensemble C d'atomes de carbone à l'état d'hybridation sp², ledit ensemble étant en contact avec :
   ∘ une pluralité de composés de formule (I₃) suivante :

      (Py-L₁-W)₃-C-L₂-X-Y (I₃),

      et
   ∘ une pluralité de composés de formule (II₃) suivante :

      (Py-L₁-W)₃-C-L₂-X-Z (II₃),

      avec, indépendamment de l'ensemble de carbone A à C et du composé de formule (I) à (II) :
      Py est un groupe pyrènyle ;
      L₁ un groupe C₁-C₁₂ alcane diyle ;
      W représente une liaison simple, un groupe arène diyle, un groupe hétéroarène diyle ou un groupe -O-Ar₁- où Ar₁ est un arène ou un hétéroarène ;
      L₂ est un groupe de formule (A) suivante -(L₂ₐ)ᵢ-(L_{2b})ⱼ- dans laquelle :
         i et j sont indépendamment l'un de l'autre choisis parmi 0 et 1, avec i + j = 1 ou 2 ;
         L₂ₐ est un groupe C₁-C₁₂ alcane diyle, C₂-C₁₂ alcène diyle ou C₂-C₁₂ alcyne diyle ;
         L_{2b} est un groupe arène diyle, un groupe hétéroarène diyle ou un groupe -O-
         Ar₂- où Ar₂ est un arène ou un hétéroarène ;
      X est un groupe -C(=O)N-, -C(=O)O- ou -C(=O)S- ;
      Y est un anticorps antibactérien ou une lectine;
      Z est un peptide antibactérien ;
et dans lequel les ensembles A, B et C sont tels que :
- l'ensemble A est dénué de composés de formule (II₁) ou le rapport molaire de la quantité de composés de formule (II₁) sur celle de composés de formule (I₁) est inférieur à 0,1;
- dans l'ensemble B, le rapport molaire de la quantité de composés de formule (I₂) sur celle de composés de formule (II₂) étant compris de 0,66 à 1,5, par exemple environ 1,0 ; et
- dans l'ensemble C, le rapport molaire de la quantité de composés de formule (I₃) sur celle de composés de formule (II₃) étant compris de 0,33 à 0,66, par exemple environ 0,5.

Selon un mode de réalisation particulier, ledit ensemble d'atomes de carbone à l'état d'hybridation sp² est supporté par un substrat, notamment choisi parmi les métaux, les oxydes métalliques, les verres et les polymères.

Selon un mode de réalisation particulier, R, L₁, W, n, V, L₂, X, Y et Z sont identiques dans les composés de formule (I) et (II), pour les ensembles A, B et C.

Selon un mode de réalisation particulier, R est le 1-pyrènyle ou le 2-pyrènyle, en particulier le 1-pyrènyle.

Selon un mode de réalisation particulier, L₁ un groupe C₂-C₈ alcane diyle, notamment le butane diyle.

Selon un mode de réalisation particulier, W représente un groupe -O-Ar₁- où Ar₁ est un arène ou un hétéroarène, en particulier un benzène, plus particulièrement un benzène substitué en para.

Selon un mode de réalisation particulier, i et j sont tous deux égaux à 1.

Selon un mode de réalisation particulier, L₂ₐ est un groupe C₂-C₁₂ alcyne diyle, en particulier -C=C-.

Selon un mode de réalisation particulier, L_{2b} est un groupe arène diyle, en particulier un benzène diyle, plus particulièrement un benzène diyle substitué en para.

Selon un mode de réalisation particulier, R, L₁, W, V, L₂ sont tels que définis par la formule suivante :

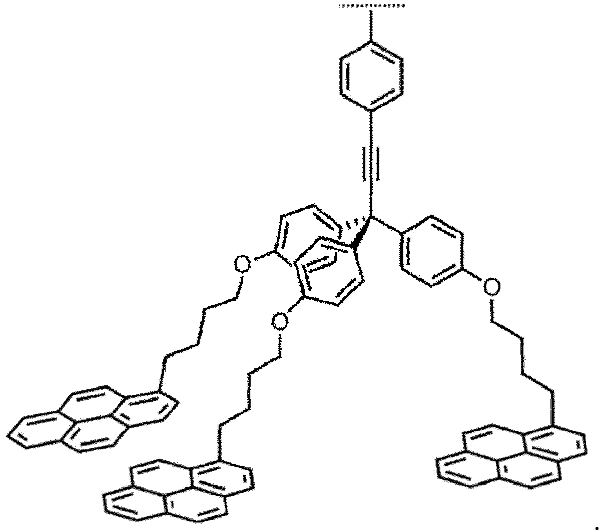

Selon un mode de réalisation particulier, Y est choisi parmi les anticorps anti bactéries gram positives, les anticorps anti bactéries gram négatives, en particulier les anticorps dirigés contre une ou plusieurs bactéries de milieu naturel et/ou d'effluent industriel, les anticorps étant plus particulièrement des anticorps anti Escherichia coli, ou en particulier les anticorps dirigés contre une ou plusieurs bactéries luminescentes, plus particulièrement Vibrio fischeri, Vibrio harveyi, Photobacterium phosphoreum, Shewanella hanedai, Shewanella woodyi, et Photorhabdus luminescens.

Les anticorps anti Escherichia coli sont bien connus de l'homme du métier. Il s'agit par exemple d'anticorps polyclonaux, notamment de chèvre ou de lapin, en particulier ceux reconnaissant de nombreux sérotypes antigéniques « O » et « K » d' Escherichia coli, notamment ceux commercialisés par LifeSpan BioSciences (par exemple sous la référence LS-C58854) ou par Abcam (par exemple sous la référence ab137967).

Il peut également s'agir d'anticorps anti Escherichia coli, de préférence monoclonaux, notamment de souris, dirigés spécifiquement contre des souches particulières d'E. coli, par exemple des souches particulièrement pathogènes comme la souche 0157. C'est notamment le cas des anticorps anti E Coli 0157 commercialisés par MyBioSource (par exemple sous la référence MBS568193).

Les anticorps anti bactéries gram positives et les anticorps anti bactéries gram négatives sont également bien connus de l'homme du métier. Il peut notamment s'agir d'anticorps monoclonaux dirigés contre les endotoxines des bactéries à Gram négatif, notamment ceux commercialisés par ThermoFisher Scientific (par exemple sous la référence MA1-10685).

Selon un mode de réalisation particulier, Y est choisi parmi les anticorps dirigés contre une ou plusieurs bactéries de milieu naturel et/ou d'effluent industriel.

Il peut s'agir de bactéries d'eau douce, notamment de bactéries du genre Aeromonas, par exemple A. hydrophila. Ainsi, Y est par exemple un anticorps anti A. hydrophila (Aquac. Res. 48 2055-63).

Il peut s'agir de bactéries marines, notamment de bactéries Photobacterium phosphoreum (Loic Recoules. Biocapteur pour la surveillance de la qualité de l'eau : Application aux eaux pluviales et de stations d'épurations. Micro et nanotechnologies/Microélectronique. Université Paul Sabatier - Toulouse III, 2015), ou de bactéries Aliivibrio fischeri (également appelée Vibrio fischeri) (ibid.). Ainsi, Y est par exemple un anticorps anti P. phosphoreum ou anti V. fischeri (cf. par exemple Test Microtox normalisé ISO 11348, 2009), notamment un anticorps polyclonal de lapin anti-vibrio marqué à la peroxydase de raifort (Horseradish peroxidase labelled anti-vibrio rabbit polyclonal antibody, Laczka OF et al., PLoS ONE 9(10), e108387.

Des bactéries d'eaux douces, marines et/ou usées peuvent également être des bactéries Escherichia coli (Loic Recoules, ibid.), par exemple des bactéries Escherichia coli portant le gène luxCDABE (issu de A. fisheri). Ainsi, Y est par exemple un anticorps anti E. coli, par exemple un anticorps polyclonal non conjugué de lapin anti E. coli (Jôgi E, Int. J. Environ. Anal. Chem. 1-12).

Selon un mode de réalisation particulier, Y est choisi parmi les anticorps dirigés contre une ou plusieurs bactéries luminescentes, plus particulièrement Vibrio fischeri, Vibrio harveyi, Photobacterium phosphoreum, Shewanella hanedai, Shewanella woodyi, et Photorhabdus luminescens.

Selon un mode de réalisation particulier, Y est choisi parmi les lectines, en particulier celles se liant à des sucres des parois bactériennes, plus particulièrement les sucres présents sur la paroi des bactéries Gram positif.

Selon un mode de réalisation particulier, Y est la concanavaline A. Ce composé est notamment commercialisé par Sigma-Aldrich (par exemple sous la référence C5275).

Selon un mode de réalisation particulier, Z est choisi parmi les cécropines, les défensines, les magainines et les dermaseptines, Z étant notamment une cécropine.

Selon un mode de réalisation plus particulier, Z est choisi parmi les cécropines, notamment la cécropine A (SEQ ID NO :1), la cécropine B (SEQ ID NO :2), la cécropine P1 (SEQ ID NO :3), la cécropine 1 (SEQ ID NO :4) et la cécropine de Bombyx mori (SEQ ID NO :5), les défensines, notamment la défensine 6 (SEQ ID NO :6) et la défensine 5 (SEQ ID NO :7), les magainines, notamment la magainine B2 (SEQ ID NO :8) et la magainine R2 (SEQ ID NO :9) et les dermaseptines, notamment la dermaseptine H3 (SEQ ID NO :10) et la dermaseptine S1 (SEQ ID NO :11).

Les cécropines A, B et P1 sont notamment commercialisées par Sigma Aldrich.

Selon un mode de réalisation particulier, la densité en composés de formule (I) et (II) à la surface de l'ensemble d'atomes de carbone à l'état d'hybridation sp² est comprise d'environ 1 de ces composés par surface de 300 nm² à environ 1 de ces composés par surface de 2 nm², notamment environ 1 composé par surface d'environ 2,7 nm².

Selon un mode de réalisation particulier, l'invention concerne un biocapteur tel que défini précédemment pour la détermination de la toxicité de substances présentes dans un milieu aqueux.

Selon un autre aspect, l'invention concerne également un procédé de préparation d'un biocapteur tel que défini précédemment, comprenant les étapes suivantes :
(i) Mise en contact avec un premier, un deuxième, et au moins un troisième ensembles d'atomes de carbone à l'état d'hybridation sp² avec un composé de formule (III) suivante

   (R-L₁-W)n-V-L₂-X-Q (III),

   dans laquelle :
   R, L₁, W, V et L₂ sont tels que définis précédemment;
   X-Q est un groupe choisi parmi -C(=O)O-N-succinimidyle, -C(=O)-halogènure, en particulier -C(=O)-Cl, -C(=O)-N₃, -C(=O)-O-N-imidazolyle, -C(=O)-O-C(=O)-R', avec
   R' étant un C₁-C₁₂ alkyle linéaire, ramifié ou cyclique, -C(=O)-O-catécholborane, - C(=O)-O-benzotriazole, en particulier -C(=O)O-N-succinimidyle,
   pour obtenir au moins trois ensembles d'atomes de carbone à l'état d'hybridation sp² activé ;
(ii) Mise en contact :
   - du premier ensemble d'atomes de carbone à l'état d'hybridation sp² activé tel qu'obtenu à l'étape précédente avec une première composition comprenant un anticorps antibactérien ou une lectine et éventuellement un peptide antibactérien, pour obtenir un premier ensemble A d'atomes de carbone à l'état d'hybridation sp² fonctionnalisé ;
   - du deuxième ensemble d'atomes de carbone à l'état d'hybridation sp² activé tel qu'obtenu à l'étape précédente avec une deuxième composition comprenant un anticorps antibactérien ou une lectine et un peptide antibactérien, pour obtenir un deuxième ensemble B d'atomes de carbone à l'état d'hybridation sp² fonctionnalisé ;
   - du troisième ensemble d'atomes de carbone à l'état d'hybridation sp² activé tel qu'obtenu à l'étape précédente avec une troisième composition comprenant un anticorps antibactérien ou une lectine et un peptide antibactérien, pour obtenir un premier ensemble C d'atomes de carbone à l'état d'hybridation sp² fonctionnalisé.

De façon alternative, la mise en contact de l'étape (ii) peut se faire de façon séquentielle, c'est-à-dire comprendre une première mise en contact avec une composition comprenant un anticorps antibactérien ou une lectine puis une deuxième mise en contact avec une composition comprenant un peptide antibactérien, ou une première mise en contact avec une composition comprenant un peptide antibactérien puis une deuxième mise en contact avec une composition comprenant un anticorps antibactérien ou une lectine.

Tous les modes de réalisation définis plus haut à propos du biocapteur à l'état d'hybridation sp² (fonctionnalisé) s'appliquent également ici, seuls ou en combinaison.

Selon un mode de réalisation particulier, la mise en contact de l'étape (i) se fait à l'aide d'une solution du composé de formule (III) dans un solvant.

Selon un mode de réalisation plus particulier, ledit solvant est choisi parmi les solvants apolaires aprotiques, notamment le tétrahydrofurane, le n-hexane, le cyclohexane, le 1,4-dioxane, le toluène, le diéthyl éther, l'acétate d'éthyle, le dichlorométhane, en particulier le tétrahydrofurane.

Selon un mode de réalisation plus particulier, le composé de formule (III) est présent dans la solution à une concentration comprise de 1 nM à 100 µM, par exemple à environ 1µM.

Selon un mode de réalisation particulier, la mise en contact de l'étape (i) se fait pendant une durée de 10 secondes à 300 secondes, par exemple environ 60 secondes.

Selon un mode de réalisation particulier, l'étape (i) est suivie, préalablement à l'étape (ii) d'une étape de lavage de l'ensemble d'atomes de carbone à l'état d'hybridation sp² activé, en particulier à l'eau.

Selon un mode de réalisation particulier, la mise en contact de l'étape (ii) se fait à l'aide d'une solution de l'anticorps antibactérien ou de la lectine et du peptide antibactérien dans un solvant.

Selon un mode de réalisation plus particulier, ledit solvant est choisi parmi les solutions salines tamponnées au phosphate, notamment le PBS 1X.

Selon un mode de réalisation plus particulier, l'anticorps antibactérien ou la lectine et le peptide antibactérien sont présents dans la solution à une concentration totale comprise de 10 pM à 10 µM, par exemple à environ 10 nM.

Selon un mode de réalisation particulier, la mise en contact de l'étape (ii) se fait pendant une durée de 10 minutes à 100 minutes, par exemple environ 30 minutes.

Selon un mode de réalisation particulier, l'étape (ii) est suivie d'une étape de lavage de l'ensemble d'atomes de carbone à l'état d'hybridation sp² fonctionnalisé, en particulier à l'aide d'une solution saline tamponnée au phosphate, notamment le PBS 1X.

Selon un mode de réalisation particulier, lesdits ensembles d'atomes de carbone à l'état d'hybridation sp2 sont supportés, indépendamment ou non, par un substrat, notamment choisi parmi les métaux, les oxydes métalliques, les verres et les polymères.

Selon un mode de réalisation particulier, lesdits ensembles d'atomes de carbone à l'état d'hybridation sp2 sont formés sur la surface dudit support, préalablement à l'étape (i).

Selon un autre mode de réalisation particulier, lesdits ensembles d'atomes de carbone à l'état d'hybridation sp2 sont formés, comme notamment bien connu de l'homme du métier, puis transférés sur la surface dudit support, préalablement à l'étape (i).

A titre d'exemple pour le graphène, lors d'un transfert par voie humide, la couche de cuivre ayant servi à faire croître le graphène peut notamment être dissoute à la surface d'un bain de gravure du cuivre (liquide) puis le graphène est mis en contact avec la surface dudit support.

Egalement à titre d'exemple pour le graphène, lors d'un transfert par voie sèche, le graphène peut notamment être détaché du substrat cuivre de croissance par ajout d'un polymère (par exemple le parylène PDMS), puis l'ensemble polymère+graphène est apposé sur la surface désirée par action mécanique (peeling mécanique). Le polymère étant ensuite retiré.

Selon un autre aspect, l'invention concerne également l'utilisation d'un biocapteur tel que défini précédemment pour la détermination de la toxicité de substances présentes dans un milieu aqueux.

Tous les modes de réalisation définis plus haut à propos du biocapteur comprenant les ensembles A, B et C d'atomes de carbones à l'état d'hybridation sp² (fonctionnalisés) s'appliquent également ici, seuls ou en combinaison.

Selon un autre aspect, l'invention concerne également un procédé de détermination de la toxicité de substances présentes dans un milieu aqueux, comprenant les étapes suivantes :
a) La mise en contact des ensembles A, B et C d'atomes de carbone à l'état d'hybridation sp² du biocapteur tels que définis précédemment, avec des bactéries susceptibles d'être reconnues par les anticorps antibactérien Y ou d'interagir avec les lectines Y desdits ensembles pour obtenir des ensembles sur lesquels lesdites bactéries sont immobilisées ;
b) La mise en contact des ensembles tels qu'obtenus à l'étape a) avec ledit milieu aqueux ;
c) L'évaluation de la viabilité desdites bactéries sur les ensembles tels qu'obtenus à l'étape b).

Selon un mode de réalisation particulier, ledit procédé est un procédé in situ.

Selon un mode de réalisation particulier, le milieu aqueux est un milieu aquatique naturel, en particulier un milieu aquatique constitué ou comprenant de l'eau douce, de préférence choisi parmi une rivière, un lac, un fleuve et un canal.

Selon un mode de réalisation particulier, le milieu aqueux est un effluent industriel.

La mise en contact selon l'étape b) peut par exemple se faire en plaçant les ensembles tels qu'obtenus à l'étape a) dans une enceinte qui communique avec le milieu extérieur, notamment via une membrane filtrante. Cette membrane filtrante a par exemple des pores dont la taille est comprises de 0,1 µm à 0,5µm.

Selon un mode de réalisation particulier, la mise en contact de l'étape b) est mise en oeuvre pendant une durée comprise de 1 à 20 minutes, par exemple de 15 minutes environ. Cette durée est susceptible de permettre la diffusion dans le milieu aqueux en contact avec lesdits ensembles tels qu'obtenus à l'étape a). Dans le cadre de l'exemple donné dans le paragraphe précédent, cette durée est susceptible de permettre que la diffusion se fasse et que les concentrations soient identiques de chaque côté de la membrane. Optionnellement, un système de circulation fluidique peut être utilisé pour forcer la diffusion à se faire plus rapidement.

Selon un mode de réalisation particulier, l'évaluation selon l'étape c) est répétée.

Selon un mode de réalisation plus particulier, l'évaluation selon l'étape c) est répétée de façon à ce que la durée séparant deux évaluations successives soit comprise de 1 minute à 30 minutes, par exemple de 15 minutes environ.

Selon un mode de réalisation particulier, la durée totale de contact entre le milieu aqueux et les ensembles tels qu'obtenus à l'étape a), correspondant en particulier à la durée totale de l'étape b) et de la ou les étapes c), est au plus comprise de 2 à 48 heures, en particulier au plus comprise de 15 à 24 heures.

Selon un mode de réalisation particulier, l'évaluation selon l'étape c) se fait par mesure de la masse desdites bactéries. Cette mesure se fait alors pour chacun des ensembles A, B et C.

A titre d'exemple, une première évaluation est réalisée selon l'étape c) et constitue une mesure de référence. L'étape c) est répétée, en particulier de façon à réaliser des mesures toutes les 15 minutes environ. Si par exemple la masse de bactéries sur l'ensemble A augmente, la masse de bactéries sur l'ensemble B reste stable, et la masse de bactéries sur l'ensemble C diminue légèrement, il peut en particulier être conclu que le milieu aqueux étudié n'est pas pollué. Au contraire, si la masse de bactéries sur l'ensemble A reste stable ou diminue, la masse de bactéries sur l'ensemble sur B diminue légèrement ou fortement, et sur la masse de bactéries sur l'ensemble C diminue fortement, notamment jusqu'à ce qu'il n'y ait plus de bactéries, il peut en particulier être conclu à une pollution du milieu aqueux. Si ce dernier cas est celui observé dès la deuxième mesure (la mesure suivant la mesure de référence), il peut en particulier être conclu qu'il s'agit d'une pollution aigüe. Si ce dernier cas est n'est observé qu'après la deuxième mesure (c'est-à-dire après plus de 30 minutes après la mesure de référence, il peut en particulier être conclu qu'il s'agit d'une pollution chronique.

Selon un mode de réalisation particulier, les substances sont des polluants, en particulier choisis parmi les polluants chimiques, physiques ou biologiques,
les polluants chimiques étant de préférence choisis parmi les hydrocarbures liquides, les détersifs et/ou tensioactifs, les plastifiants, les pesticides, les matières eutrophisantes, les métaux lourds, les médicaments, les cosmétiques et les produits industriels ;
les polluants physiques étant de préférence choisis parmi la chaleur, le bruit, la radioactivité et la lumière ;
les polluants biologiques étant de préférence choisis parmi les toxines algales, les germes pathogènes et les parasites.

Selon un mode de réalisation particulier préféré, les substances sont des polluants chimiques.

Selon un mode de réalisation particulier, le milieu aqueux contient une ou plusieurs bactéries, et les groupes Y sont choisis parmi les anticorps dirigés contre cette ou ces plusieurs bactéries. Ainsi, dans ce mode de réalisation particulier, les groupes Y sont choisis parmi les anticorps dirigés contre une ou des bactéries susceptibles d'être contenues dans le milieu aqueux pour lequel la mesure de la présence de substances toxiques est souhaitée.

Selon un mode de réalisation particulier, les groupes Y sont choisis parmi les anticorps dirigés contre une ou plusieurs bactéries luminescentes, les bactéries de l'étape a) sont ladite ou lesdites plusieurs bactéries luminescentes, et l'évaluation de la viabilité desdites bactéries selon l'étape c) se fait par irradiation à l'aide d'une lampe UV puis mesure de la fluorescence, notamment à l'aide d'une caméra.

Selon un mode de réalisation particulier, l'évaluation de la viabilité des bactéries selon l'étape c) se fait par mesure de la masse desdits ensembles A, B et C.

Selon un mode de réalisation particulier, l'évaluation de la viabilité des bactéries selon l'étape c) se fait à l'aide de capteurs optomécaniques.

Selon un mode de réalisation plus particulier, l'évaluation de la viabilité des bactéries selon l'étape c) se fait à l'aide de capteurs optomécaniques comportant un support, au moins un guide d'onde, au moins un résonateur optique suspendu au support, ledit résonateur optique étant couplé optiquement au guide d'onde, au moins un résonateur mécanique suspendu au support, ledit résonateur mécanique et ledit résonateur optique étant couplés, ledit résonateur mécanique étant configuré pour vibrer en mode de volume et comportant au moins une face s'étendant dans le plan du capteur et configurée pour recevoir des molécules de ladite espèce donnée, au moins ladite face comportant respectivement l'ensemble A, l'ensemble B, et le au moins un ensemble C respectivement, ledit résonateur mécanique présentant une dimension faible dans une direction normale au plan du capteur par rapport aux dimensions de ladite face.

Selon un mode de réalisation particulier, l'évaluation de la viabilité des bactéries selon l'étape c) se fait à l'aide d'une microbalance à quartz.

Par exemple, chaque ensemble A, B et C peut être déposé sur une microbalance à quartz, et la fréquence de résonnance lue à un temps déterminé, par exemple toutes les minutes. Une augmentation de la fréquence de résonnance correspond à une augmentation de la masse, et ainsi à une croissance bactérienne, et donc à un milieu susceptible d'être favorable à la croissance bactérienne car exempt de polluant. Par ailleurs, une fréquence stable correspond à une masse constante, c'est-à-dire que les bactéries étudiées ne sont ni en croissance, ni en décroissance, car les bactéries qui meurent sont par exemple remplacées par les nouvelles. Enfin, si la fréquence de résonnance diminue, cela signifie très généralement que la masse de l'ensemble diminue, et donc que les bactéries meurent dans le milieu. Ceci est susceptible de signifier, en particulier si cela concerne l'ensemble A et éventuellement l'ensemble B, qu'il y a présence de polluant.

### Définitions

Tel qu'on l'utilise dans la présente description, le terme « environ » se réfère à un intervalle de valeurs de ± 10 % d'une valeur spécifique. A titre d'exemple, l'expression « environ 20 » comprend les valeurs de 20 ± 10 %, soit les valeurs de 18 à 22.

Au sens de la présente description, les pourcentages se réfèrent à des pourcentages en masse par rapport à la masse totale de la formulation, sauf indication contraire.

Tel qu'on l'entend ici, les plages de valeur sous forme de « x-y » ou « de x à y » ou « entre x et y » incluent les bornes x et y ainsi que les entiers compris entre ces bornes. A titre d'exemple, « 1-5 », ou « de 1 à 5 » ou « entre 1 et 5 » désignent les entiers 1, 2, 3, 4 et 5. Les modes de réalisations préférés incluent chaque entier pris individuellement dans la plage de valeur, ainsi que toute sous-combinaison de ces entiers. A titre d'exemple, les valeurs préférées pour « 1-5 » peuvent comprendre les entiers 1, 2, 3, 4, 5, 1-2, 1-3, 1-4, 1-5, 2-3, 2-4, 2-5, etc.

Tel qu'il est utilisé ici, le terme "alkyle" désigne un groupe alkyle à chaîne linéaire ou ramifiée ayant le nombre d'atomes de carbone indiqué avant ledit terme, notamment 1 à 8 atomes de carbone, tel que méthyle, éthyle, propyle, isopropyle, butyle, isobutyle, sec-butyle, tert-butyle, pentyle, isoamyle, néopentyle, 1-éthylpropyle, 3-méthylpentyle, 2,2-diméthylbutyle, 2,3-diméthylbutyle, hexyle, octyle, etc. Ainsi, une expression telle que "alkyle en C1-C4" désigne un radical alkyle contenant de 1 à 4 atomes de carbone. Il en est de même pour le terme « alcane ». Tel qu'utilisé ici, le terme "arène" désigne un système cyclique aromatique hydrocarboné monoou bicyclique, substitué ou non substitué, ayant 6 à 10 atomes de carbone dans le cycle. Les exemples incluent le benzène et le naphtalène. Les arènes préférés comprennent le benzène et le naphtalène non substitués ou substitués. Sont inclus dans la définition d'"arène" les systèmes cycliques condensés, y compris, par exemple, les systèmes cycliques dans lesquels un cycle aromatique est condensé à un cycle cycloalkyle. Les exemples de tels systèmes cycliques condensés comprennent, par exemple, l'indane, l'indène et le tétrahydronaphtalène.

Tel qu'il est utilisé ici, le terme "hétéroarène" désigne un système aromatique cyclique contenant 5 à 10 atomes de carbone dans lequel un ou plusieurs atomes de carbone du cycle sont remplacés par au moins un hétéroatome tel que -O-, -N- ou -S-. Les exemples d'hétéroarènes comprennent pyrrole, furane, thiophène, pyrazole, imidazole, thiazole, isothiazole, isoxazole, oxazole, oxathiol, oxadiazole, triazole, oxatriazole, furazane, tétrazole, pyridine, pyrazine, pyrimidine, pyridazine, triazine, indole, isoindole, indazole, benzofurane, isobenzofurane, purine, quinazoline, quinoline, isoquinoline, benzoimidazole, benzothiazole, benzothiophène, thianaphtène, benzoxazole, benzisoxazole, cinnoline, phtalazine, naphtyridine et quinoxaline. Sont inclus dans la définition d'"hétéroarène" les systèmes cycliques condensés, y compris, par exemple, les systèmes cycliques dans lesquels un cycle aromatique est condensé à un cycle hétérocycloalkyle. Les exemples de tels systèmes cycliques fusionnés comprennent, par exemple, le phtalamide, l'anhydride phtalique, l'indoline, l'isoindoline, la tétrahydroisoquinoline, le chromane, l'isochromane, le chromène et l'isochromène.

**[Table 1]**

| | |
|---|---|
| SEQ ID NO :1 | Lys Trp Lys Leu Phe Lys Lys Ile Glu Lys Val Gly Gln Asn Ile Arg Asp Gly Ile Ile Lys Ala Gly Pro Ala Val Ala Val Val Gly Gln Ala Thr Gln Ile Ala Lys |
| SEQ ID NO :2 | Lys Trp Lys Val Phe Lys Lys Ile Glu Lys Met Gly Arg Asn Ile Arg Asn Gly Ile Val Lys Ala Gly Pro Ala Ile Ala Val Leu Gly Glu Ala Lys Ala Leu |
| SEQ ID NO :3 | Ser Trp Leu Ser Lys Thr Ala Lys Lys Leu Glu Asn Ser Ala Lys Lys rg Ile Ser Glu Gly Ile Ala Ile Ala Ile Gln Gly Gly Pro Arg |
| SEQ ID NO :4 | Met Asn Phe Asn Lys Val Phe Ile Leu Val Ala Ile Val Ile Ala Ile Phe Ala Gly Gln Thr Glu Ala Gly Trp Leu Lys Lys Ile Gly Lys Lys Ile Glu Arg Val Gly Gln His Thr Arg Asp Ala |
| | Thr Ile Gln Thr Ile Ala Val Ala Gln Gln Ala Ala Asn Val Ala Ala Thr Ala Arg Gly |
| SEQ ID NO :5 | Arg Trp Lys Ile Phe Lys Lys Ile Glu Lys Val Gly Gln Asn Ile Arg Asp Gly Ile Val Lys Ala Gly Pro Ala Val Ala Val Val Gly Gln Ala Ala Thr Ile |
| SEQ ID NO :6 | Met Arg Thr Leu Thr Ile Leu Thr Ala Val Leu Leu Val Ala Leu Gln Ala Lys Ala Glu Pro Leu Gln Ala Glu Asp Asp Pro Leu Gln Ala Lys Ala Tyr Glu Ala Asp Ala Gln Glu Gln Arg Gly Ala Asn Asp Gln Asp Phe Ala Val Ser Phe Ala Glu Asp Ala Ser Ser Ser Leu Arg Ala Leu Gly Ser Thr Arg Ala Phe Thr Cys His Cys Arg Arg Ser Cys Tyr Ser Thr Glu Tyr Ser Tyr Gly Thr Cys Thr Val Met Gly Ile Asn His Arg Phe Cys Cys Leu |
| SEQ ID NO :7 | Met Arg Thr Ile Ala Ile Leu Ala Ala Ile Leu Leu Val Ala Leu Gln Ala Gln Ala Glu Ser Leu Gln Glu Arg Ala Asp Glu Ala Thr Thr Gln Lys Gln Ser Gly Glu Asp Asn Gln Asp Leu Ala Ile Ser Phe Ala Gly Asn Gly Leu Ser Ala Leu Arg Thr Ser Gly Ser Gln Ala Arg Ala Thr Cys Tyr Cys Arg Thr Gly Arg Cys Ala Thr Arg Glu Ser Leu Ser Gly Val Cys Glu Ile Ser Gly Arg Leu Tyr Arg Leu Cys Cys Arg |
| SEQ ID NO :8 | Gly Ile Gly Lys Phe Leu His Ser Ala Gly Lys Phe Gly Lys Ala Phe Leu Gly Glu Val Met Lys Ser |
| SEQ ID NO :9 | Gly Ile Lys Glu Phe Ala His Ser Leu Gly Lys Phe Gly Lys Ala Phe Val Gly Gly Ile Leu Asn Gln |
| SEQ ID NO :10 | Met Ala Phe Leu Lys Lys Ser Leu Phe Leu Val Leu Phe Leu Gly Met Val Ser Leu Ser Ile Cys Glu Glu Glu Lys Arg Glu Asn Glu Asp Glu Glu Leu Gln Glu Asp Asp Glu Gln Ser Glu Met Lys Arg Gly Leu Trp Ser Thr Ile Lys Asn Val Gly Lys Glu Ala Ala Ile Ala Ala Gly Lys Ala Ala Leu Gly Ala Leu |
| SEQ ID NO :11 | Met Asp Ile Leu Lys Lys Ser Leu Phe Leu Val Leu Phe Leu Gly Leu Val Ser Leu Ser Ile Cys Glu Glu Glu Lys Arg Glu Asn Glu Asp Glu Glu Lys Gln Glu Asp Asp Glu Gln Ser Glu Met Lys Arg Ala Leu Trp Lys Thr Met Leu Lys Lys Leu Gly Thr Met Ala Leu His Ala Gly Lys Ala Ala Leu Gly Ala Ala Ala Asp Thr Ile Ser Gln Gly Thr Gln |

### FIGURES

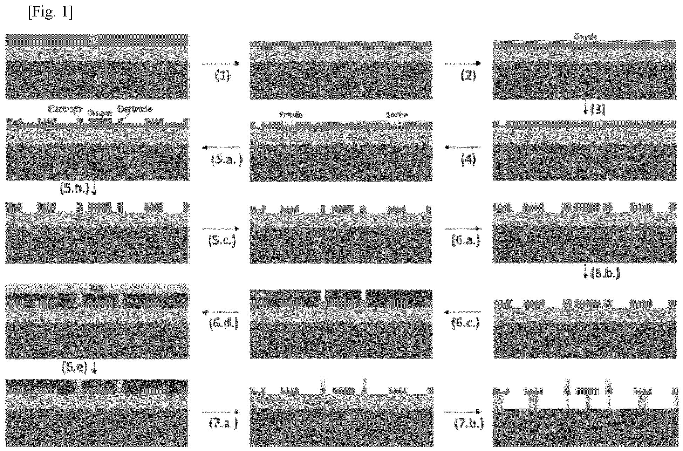

La [Fig.1] présente un protocole de fabrication des biocapteurs selon l'exemple 1.

Les étapes illustrées sont comme suit :
1. Amincissement de la couche supérieure de Si
2. Oxydation thermique
3. Marquages d'alignement pour la lithographie
4. Fabrication du réseau optique (entrée et sortie)
5. Fabrication des guides optiques, des microdisques et des électrodes.
   a) Dépôt chimique en phase vapeur à basse pression et création d'un masque dur d'oxyde de silane (SiH₄) à haute température
   b) Lithogravure de la couche supérieure de Si et élimination des résidus
   c) Elimination du SiH₄ et des oxydes natifs
6. Fabrication des électrodes en AlSi
   a) Seconde oxydation thermique
   b) Dopage localisé de Si
   c) Dépôt chimique en phase vapeur d'oxyde de SiH₄, aplanissement et modelage (oxyde de SiH₄)
   d) Dépôt en phase vapeur de AlSi
   e) Aplanissement chimico-mécanique de AlSi
7. Formation du socle du microdisque
   a) Gravure à la vapeur d'acide fluorhydrique des couches de SiH₄ et des oxydes natifs
   b) Gravure à la vapeur d'acide fluorhydrique de la couche d'oxyde (BOX).

### EXEMPLES

### Exemple 1 : Fabrication de biocapteurs optomécaniques surmontés de graphène fonctionnalisé selon l'invention

La fabrication de ces biocapteurs en graphène fonctionnalisés se déroule en 3 étapes.

### 1. Fabrication des biocapteurs

Un protocole de fabrication des biocapteurs est présenté à la figure 1 (Microdisques optomécaniques résonants en silicium pour la détection biologique en milieu liquide, thèse de Maxime HERMOUET soutenue le 26.03.2019, Communauté Université Grenoble Alpes).

### 2. Fabrication et dépôt de graphène sur les microdisques des biocapteurs

La fabrication et le dépôt du graphène sont généralement effectués comme suit. Ce dépôt est généralement effectué entre les étapes 7a) et 7 b) telles que décrites dans la figure 1.

Le protocole est présenté ci-dessous (par exemple en ligne avec Courtin Jules. Hétérostructure graphène / silicium : de la formation de l'interface aux propriétés de transport électronique. Physique. Université Rennes 1, 2020) :
1. La fabrication de graphène peut se faire au travers d'un dépôt chimique en phase vapeur à partir d'un mélange de gaz, dont au moins un est composé de carbone, qui est chauffé jusqu'à obtention d'un plasma. Ce plasma permet ensuite le dépôt d'une couche de graphène sur du cuivre
2. Le graphène est ensuite recouvert par spin-coating d'un film PMMA (PolyMethylMethacrylAte)
3. Le cuivre est ensuite gravé par une solution de FeCl₃ à 9%
4. Des bains successifs d'eau déionisée et de HCl à 12% permettent de réduire les pollutions induites par le bain de FeCl₃
5. L'empilement PMMA/graphène est ensuite transféré sur la surface de silicium du microdisque du biocapteur
6. Des bains d'acétone et d'isopropranolol pendant 1h à température ambiante permettent d'éliminer un maximum de résidus de PMMA.
7. Optionnellement, une étape d'enduction de résine photosensible / de photolithographie / ou de gravure RIE permet de retirer le graphène aux endroits non souhaités (c'est-à-dire par exemple en dehors de disques)

### 3. Fonctionnalisation des couches de graphène

La fonctionnalisation est réalisée par greffage du tripode de l'invention sur la monocouche de graphène, puis traitement par des anticorps et/ou peptide antimicrobien, comme défini précédemment, et comme suit :

### Surface favorable au développement de bactéries - ensemble A

Un rapport molaire peptide antimicrobien / anticorps inférieur à 0,1, par exemple 100% d'anticorps

### Surface bactériostatique - ensemble B

Un rapport molaire anticorps / peptide antimicrobien compris de 0,66 à 1,5, par exemple :
50% molaire d'anticorps
50% molaire de peptide antimicrobien

### Surface bactéricide - ensemble C

Un rapport molaire anticorps / peptide antimicrobien compris de 0,33 à 0,66, par exemple :
25% molaire d'anticorps
75% molaire de peptide antimicrobien

### Exemple 2 : Utilisation des biocapteurs

Phase optionnelle de test :
Afin de vérifier la survie des bactéries dans les conditions de stress désirées, les biocapteurs développés sont testés, notamment comme suit dans des matrices de plus en plus complexe. Pour ce faire, les solutions suivantes sont injectées dans le système microfluidique du biocapteur de l'exemple 1:
1) solution tampon biologique/physiologique utilisée en microbiologie (Ringer, Phosphate-Buffered Saline (PBS)) ;
2) eaux naturelles filtrées (eau douce ou marine) ;
3) eaux naturelles non filtrées (eau douce ou marine) ;
4) et eaux usées.

Utilisation proprement dite :
Les bactéries utilisées dans les biocapteurs sont notamment choisies en fonction du choix des eaux douces/marines/eaux usées étudiées.

La survie des bactéries est identifiable au travers des images obtenues par la caméra, pour les bactéries bioluminescentes, et/ou par la variabilité des signaux optiques et électriques (MEMS-OMNR : microsystème électromécanique -nanorésonateur optomécanique ).

Le temps nécessaire à l'observation d'un début de décroissance bactérienne avec les solutions tampons renseigne sur la « durée de validité » des capteurs.

Les résultats observables (par exemple bactéries viables, bactéries viables mais qui ne se multiplient pas, bactéries non viables) en fonction des conditions de stress présentées ci-dessous :
- stress minimum : surface100% anticorps / 0% peptides antimicrobiens ;
- conditions bactériostatiques : 50% anticorps / 50% peptides antimicrobiens ;
- conditions bactéricides (stress très élevé voire maximal) : 25% anticorps / 75% peptides antimicrobiens.
permettent une évaluation des risques éco-toxicologiques. Par exemple, plus les eaux analysées ont la capacité d'impacter les bactéries soumises à des niveaux de stress négligeables, plus les risques éco-toxicologiques pourront être considérés comme non négligeables.

Détails d'utilisation du capteur :
On installe le capteur dans le milieu aqueux à mesurer, le système étant partiellement ou totalement immergé.

Les différents ensembles du ou des capteurs sont installés dans une enceinte qui communique avec le milieu extérieur via une membrane filtrante (taille de pore entre 0.1 µm et 0,5µm) et on laisse quelques minutes le temps que la diffusion se fasse et que les concentrations soient identiques de chaque côté de la membrane. Optionnellement, on peut avoir un système de circulation fluidique pour forcer la diffusion à se faire plus rapidement.

On prend une 1^{ère} mesure de chacun des ensembles, laquelle correspond à une mesure de référence.

Puis, de nouvelles mesures sont faites toutes les 15 minutes.

Deux cas peuvent être distingués :
Cas 1 : pas de pollution dans le milieu : la masse de bactéries sur ensemble A augmente, la masse de bactéries sur B reste stable et sur C diminue légèrement (surface bactéricide donc les bactéries vont généralement mourir).
Cas 2 : Pollution : la masse sur A reste stable ou diminue, sur B diminue légèrement ou fortement et sur C diminue fortement jusqu'à ce qu'il n'y ait plus de bactéries.
Si le cas 2 est observé dès la 2^{ème} mesure (15 minutes après équilibre), il s'agit généralement d'une pollution aigue.
Si le cas 2 n'est observé qu'après plus de 30 minutes, il s'agit généralement d'une pollution chronique.

## Revendications

1. Biocapteur comprenant :
- un premier ensemble A d'atomes de carbone à l'état d'hybridation sp², ledit ensemble comprenant, au contact avec lesdits atomes de carbone à l'état d'hybridation sp² :
∘ une pluralité de composés de formule (I₁) suivante :
(R-L₁-W)ₙ-V-L₂-X-Y (I₁),
et
∘ éventuellement, une pluralité de composés de formule (II₁) suivante :
(R-L₁-W)ₙ-V-L₂-X-Z (II₁),
- un deuxième ensemble B d'atomes de carbone à l'état d'hybridation sp², ledit ensemble comprenant, au contact avec lesdits atomes de carbone à l'état d'hybridation sp² :
∘ une pluralité de composés de formule (I₂) suivante :
(R-L₁-W)ₙ-V-L₂-X-Y (I₂),
et
∘ une pluralité de composés de formule (II₂) suivante :
(R-L₁-W)ₙ-V-L₂-X-Z (II₂),
- au moins un troisième ensemble C d'atomes de carbone à l'état d'hybridation sp², ledit ensemble comprenant, en contact avec lesdits atomes de carbone à l'état d'hybridation sp² :
∘ une pluralité de composés de formule (I₃) suivante :
(R-L₁-W)ₙ-V-L₂-X-Y (I₃),
et
∘ une pluralité de composés de formule (II₃) suivante :
(R-L₁-W)ₙ-V-L₂-X-Z (II₃),
avec, indépendamment de l'ensemble de carbone A à C et du composé de formule (I) à (II) :
n est égal à 1, 2, ou 3, notamment 3 ;
V représente, pour tout n, -C(H) ₃₋ₙ- ou encore:
- lorsque n est égal à 2, -C(H)=, C étant dans ce cas lié à L₂ par une liaison double ;
- lorsque n est égal à 1, -C=, C étant dans ce cas lié à L₂ par une liaison triple ;
R est un hydrocarbure aromatique comprenant de 2 à 6 cycles aromatiques condensés; L₁ un groupe C₁-C₁₂ alcane diyle ;
W représente une liaison simple, un groupe arène diyle, un groupe hétéroarène diyle ou
un groupe -O-Ar₁- où Ar₁ est un arène ou un hétéroarène ;
L₂ est un groupe de formule (A) suivante -(L₂ₐ)ᵢ-(L_{2b})ⱼ- dans laquelle :
i et j sont indépendamment l'un de l'autre choisis parmi 0 et 1, avec i + j = 1 ou 2 ;
L₂ₐ est un groupe C₁-C₁₂ alcane diyle, C₂-C₁₂ alcène diyle ou C₂-C₁₂ alcyne diyle ;
L_{2b} est un groupe arène diyle, un groupe hétéroarène diyle ou un groupe -O-
Ar₂- où Ar₂ est un arène ou un hétéroarène ;
X est un groupe -C(=O)N-, -C(=O)O- ou -C(=O)S- ;
Y est un anticorps antibactérien ou une lectine se liant à des sucres des parois bactériennes
Z est un peptide antibactérien ;
et dans lequel les ensembles A, B et C sont tels que :
- l'ensemble A est dénué de composés de formule (II₁) ou le rapport molaire de la quantité de composés de formule (II₁) sur celle de composés de formule (I₁) est inférieur à 0,1;
- dans l'ensemble B, le rapport molaire de la quantité de composés de formule (I₂) sur celle de composés de formule (II₂) étant compris de 0,66 à 1,5, par exemple environ 1,0 ; et
- dans l'ensemble C, le rapport molaire de la quantité de composés de formule (I₃) sur celle de composés de formule (II₃) étant compris de 0,33 à 0,66, par exemple environ 0,5.

2. Biocapteur selon la revendication 1, comprenant :
- au moins deux ensembles B d'atomes de carbone à l'état d'hybridation sp², pour lesquels le rapport molaire de la quantité de composés de formule (I₂) sur celle de composés de formule (II₂), étant compris de 0,66 à 1,5, diffère ; et/ou
- au moins deux ensembles C d'atomes de carbone à l'état d'hybridation sp², pour lesquels le rapport molaire de la quantité de composés de formule (I₃) sur celle de composés de formule (II₃), étant compris de 0,33 à 0,66, diffère.

3. Biocapteur selon l'une quelconque des revendications précédentes, dans lequel lesdits ensembles A, B et C d'atomes de carbone à l'état d'hybridation sp² sont indépendamment choisis parmi le graphène, l'oxyde de graphène, le graphite, les nanotubes de carbones, les fullerènes et les fullerites, lesdits ensembles étant en particulier du graphène, plus particulièrement sous la forme de feuille ou de flocons.

4. Biocapteur selon l'une quelconque des revendications précédentes, dans lequel R, L₁, W, n, V, L₂, X, Y et Z sont identiques dans les composés de formule (I) et (II), pour les ensembles A, B et C.

5. Biocapteur selon l'une quelconque des revendications précédentes, dans lequel :
- R est le 1-pyrènyle ou le 2-pyrènyle, en particulier le 1-pyrènyle ;
- L₁ un groupe C₂-C₈ alcane diyle ; et/ou
- W représente un groupe -O-Ar₁- où Ar₁ est un arène ou un hétéroarène, en particulier un benzène ; et/ou
- i et j sont égaux à 1 ; et/ou
- L₂ₐ est un groupe C₂-C₁₂ alcyne diyle, en particulier -C=C- ; et/ou
- L_{2b} est un groupe arène diyle, en particulier un benzène diyle ; et/ou
- R, L₁, W, V et L₂ sont tels que définis par la formule suivante :

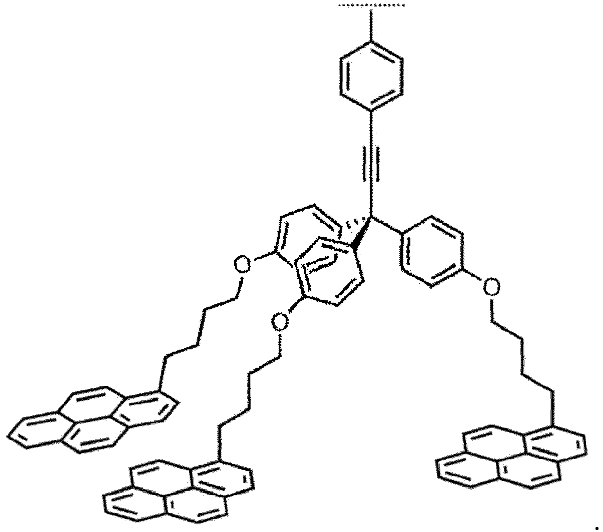

6. Biocapteur selon l'une quelconque des revendications précédentes, dans lequel :
- Y est choisi parmi les anticorps anti bactéries gram positives, les anticorps anti bactéries gram négatives, en particulier les anticorps dirigés contre une ou plusieurs bactéries de milieu naturel et/ou d'effluent industriel, les anticorps étant plus particulièrement des anticorps anti Escherichia coli, ou en particulier les anticorps dirigés contre une ou plusieurs bactéries luminescentes, plus particulièrement Vibrio fischeri, Vibrio harveyi, Photobacterium phosphoreum, Shewanella hanedai, Shewanella woodyi, et Photorhabdus luminescens ;
- Z est choisi parmi les cécropines, les défensines, les magainines et les dermaseptines, Z étant notamment une cécropine.

7. Biocapteur selon l'une quelconque des revendications précédentes, dans lequel la densité en composés de formule (I) et (II) à la surface des ensembles d'atomes de carbone à l'état d'hybridation sp² est comprise d'environ 1 de ces composés par surface de 300 nm² à environ 1 de ces composés par surface de 2 nm², notamment environ 1 composé par surface d'environ 2,7 nm².

8. Utilisation d'un biocapteur selon l'une quelconque des revendications 1 à 7, pour la détermination de la toxicité de substances présentes dans un milieu aqueux.

9. Procédé de détermination de la toxicité de substances présentes dans un milieu aqueux, comprenant les étapes suivantes :
a) La mise en contact des ensembles A, B et C d'atomes de carbone à l'état d'hybridation sp² du biocapteur selon l'une quelconque des revendications 1 à 7, avec des bactéries susceptibles d'être reconnues par les anticorps antibactérien Y ou d'interagir avec les lectines Y desdits ensembles pour obtenir des ensembles sur lesquels lesdites bactéries sont immobilisées ;
b) La mise en contact des ensembles tels qu'obtenus à l'étape a) avec ledit milieu aqueux ;
c) L'évaluation de la viabilité desdites bactéries sur les ensembles tels qu'obtenus à l'étape b).

10. Procédé selon la revendication 9, dans lequel :
- les groupes Y sont choisis parmi les anticorps dirigés contre une ou des bactéries susceptibles d'être contenues dans le milieu aqueux pour lequel la mesure de la présence de substances toxiques est souhaitée ; ou
- les groupes Y sont choisis parmi les anticorps dirigés contre une ou plusieurs bactéries luminescentes, les bactéries de l'étape a) sont ladite ou lesdites plusieurs bactéries luminescentes, et l'évaluation de la viabilité desdites bactéries selon l'étape c) se fait par irradiation à l'aide d'une lampe UV puis mesure de la fluorescence, notamment à l'aide d'une caméra.

11. Procédé selon l'une quelconque des revendications 9 à 10, dans lequel l'évaluation de la viabilité des bactéries selon l'étape c) se fait par mesure de la masse desdits ensembles A, B et C.
